# EUROPEAN PATENT APPLICATION

(11) **EP 4 491 176 A1**
(43) Date of publication of application: **15.01.2025**
(21) Application number: 24181109.0
(22) Date of filing: 10.06.2024
(51) Int. Cl.: A61K 9/20

(54) **A FILM COATED TABLET COMPRISING BRIVARACETAM**

(30) Priority: 14.07.2023 TR 202308263
(71) Applicant: Sanovel Ilac Sanayi ve Ticaret A.S., Sisli - Istanbul (TR)
(72) Inventor: SUNEL, Fatih, Istanbul (TR); BILGEHAN ATAK, Fadime, Istanbul (TR); ATAMAN, Seval, Istanbul (TR); DERELI, Selin, Istanbul (TR)
(74) Representative: Genç Ilhan, Oznur

(57) **Abstract**

The present invention relates to a film coated tablet comprising brivaracetam and at least one pharmaceutically acceptable excipient wherein microcrystalline cellulose as diluent is present and the amount of disintegrant is 4.0% to 10.0% by weight in the total composition. Furthermore, the tablet is also obtained by using an effective process which is simple, rapid, cost effective, time-saving and industrially convenient process.

## Description

### Field of the Invention

The present invention relates to a film coated tablet comprising brivaracetam and at least one pharmaceutically acceptable excipient wherein microcrystalline cellulose as diluent is present and the amount of disintegrant is 4.0% to 10.0% by weight in the total composition. Furthermore, the tablet is also obtained by using an effective process which is simple, rapid, cost effective, time-saving and industrially convenient process.

### Background of the Invention

The chemical name of brivaracetam is (2S)-2-[(4R)-2-oxo-4-propyl-pyrrolidin-1-yl]butanamide and its chemical structure is shown in the Formula I.

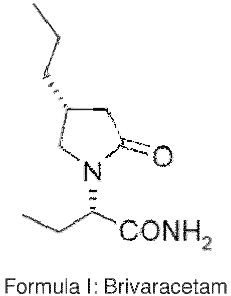

The European Medicines Agency (EMA) and the US Food and Drug Administration (FDA) approved brivaracetam as an adjunctive therapy drug for the treatment of partial seizures with or without secondary generalized seizures of epilepsy patients of 16-year-old and older, under the trade name Briviact^{®}. The drug is a derivative of levetiracetam and belongs to the 3rd generation of antiepileptic drug. Clinical trials evaluated the efficacy and safety of Brivaracetam (5, 20, 50 and 150 mg per day) in the adjunctive treatment of adult patients (16-65 years).

Brivaracetam is highly soluble and is Class I according to the Biopharmaceutical Classification System (BCS). The active substance is a white to off-white non-hygroscopic crystalline solid. Additionally, brivaracetam is an extremely viscous compound (adhesive capacity).

We have found that Brivaracetam is extremely prone to sticking and picking due to high adhesion during the preparation process, which brings great problems to the appearance and content uniformity of the final product. Also, during the process, problems such as flowability and compressibility appear. These problems lead to both solubility and stability problems.

Patent CN102292071A discloses that the excipient "cyclodextrin" is used as a compacting agent to produce brivaracetam tablets, but since brivaracetam is an extremely viscous compound (adhesion ability), cyclodextrin can reduce the compaction and especially the bonding during the rolling process alleviates the risk of tablet sticking to a certain extent.

PCT Patent Publications No. WO 2010/086315 and WO 2010/094535 disclose immediate release oral pharmaceutical formulation of Brivaracetam. These patent publications discloses dissolution, sticking problem associated with Brivaracetam, which is a major challenge during the formulation development.

In the prior art, several patents have been done for the tablet sticking problem of brivaracetam. However, since brivaracetam is an active ingredient that has flowability, compressibility and stability problems in formulation, focusing only on its tablet sticking to will not yield successful results.

There still remains a need in the art to provide an improved a film coated tablet comprising brivaracetam. In the present invention, the film coated tablet is created to overcome the above problems and provides additional advantages to the relevant field of art. Other advantages and embodiments of the present invention will be clarified in the following description.

### Detailed description of the Invention

The main object of the present invention is to provide a film coated tablet comprising brivaracetam with excellent properties, such as compressibility, flowability, homogeneity, and content uniformity which overcomes the above-described problems in the prior art and have additive advantages over them.

Another object of the present invention is to provide a desired stability of the film coated tablet comprising brivaracetam.

Another object of the present invention is to provide a film coated tablet comprising brivaracetam with the desired dissolution profile.

Another object of the present invention is to provide a process for preparing a film coated tablet comprising brivaracetam. The process is free of solvent, for example dry granulation or direct compression. It is a simple, rapid, cost effective, time-saving, and industrially convenient method.

We encountered many problems while creating a formulation comprising brivaracetam, but the most important was the problem of content uniformity. Because of content uniformity problems, the finished product may show deviations in assay. It reflects that content uniformity play important role in the dissolution of the drug.

Flowability is an important parameter in tablet formulation. Especially the sticky nature (high viscosity) of the active substance caused flowability problems in formulations. The problem with flowability negatively affects the content uniformity, and the problem with the content uniformity negatively affects the dissolution profile. We have found in this invention that particularly used microcrystalline cellulose overcomes this flowability problem. In addition, the compressibility and anti-sticking effect problem was overcome with the use of disintegrant at the specified ranges. So, both the content uniformity was ensured and the dissolution profile was brought to the desired level.

According to one embodiment of this invention, a film coated tablet comprises brivaracetam and at least one pharmaceutically acceptable excipient wherein microcrystalline cellulose as diluent is present and the amount of disintegrant is 4.0% to 10.0% by weight in the total composition.

According to an embodiment of the present invention, the amount of microcrystalline cellulose is 25.0% to 50.0% by weight in the total composition.

The composition comprises a disintegrant, and the inventors have found that the use of the specified amount ranges of disintegrant in the pharmaceutical composition of the present invention has an unexpected anti-sticking effect, and used disintegrant is unexpectedly used as an anti-sticking agent for powder direct compression or dry granulation, It is beneficial to reduce the viscosity of the active ingredient and avoid sticking and punching when tableting.

Suitable disintegrants are selected from the group comprising crospovidone, croscarmellose sodium, low-substituted hydroxypropyl cellulose, sodium starch glycolate, carboxymethyl cellulose, sodium carboxymethyl cellulose, calcium carboxymethyl cellulose, sodium carboxymethyl starch, hydroxymethyl starch or mixtures thereof.

According to an embodiment of the present invention, the disintegrant is croscarmellose sodium or low-substituted hydroxypropyl cellulose or sodium starch glycolate or mixtures thereof.

According to one embodiment of this invention, the film coated tablet comprises one more diluent. Suitable diluents are selected from the group comprising lactose anhydrous, lactose, lactose monohydrate, corn starch, starch, mannitol, talc, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to an embodiment of the present invention, the diluent is talc, starch, lactose monohydrate or lactose anhydrous or mixtures thereof.

According to an embodiment of the present invention, the amount of diluents is 70.0% to 86.0% by weight in the total composition.

According to an embodiment of the present invention, the amount of brivaracetam is 5.0% to 20.0% by weight in the total composition.

As used here in, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles is finer.

According to one embodiment of this invention, brivaracetam has a d (0.9) particle size between 100 µm to 300 µm. Brivaracetam having above-described particle size provides the flowability and compressibility, also provides the improved dissolution profile.

According to one embodiment of this invention, brivaracetam has a d (0.9) particle size between 104 µm to 125 µm or between 126 µm to 138 µm or between 139 µm to 152 µm or between 153 µm to 167 µm or between 168 µm to 183 µm or between 184 µm to 198 µm or between 203 µm to 218 µm or between 219 µm to 234 µm or between 237 µm to 253 µm or between 257 µm to 268 µm or between 269 µm to 278 µm or between 283 µm to 298 µm.

According to one embodiment of this invention, brivaracetam has a d (0.1) particle size between 1 µm to 40 µm or between 4 µm to 34 µm or between 6 µm to 25 µm.

According to one embodiment of this invention, brivaracetam has a d (0.5) particle size between 8 µm to 85 µm or between 13 µm to 76 µm or between 22 µm to 65 µm or between 27 µm to 58 µm.

According to one embodiment of this invention, brivaracetam has a d (0.1) particle size between 1 µm to 40 µm and a d (0.5) particle size between 8 µm to 85 µm and a d (0.9) particle size between 100 µm to 300 µm.

In general terms, excipients provided in a composition may positively or negatively influence the physicochemical and pharmacokinetic properties, e.g. the solubility, stability, absorption, bioavailability of an active agent. For this reason, the excipients which accompany an active agent have to be selected in a careful and conscious manner.

According to an embodiment of the present invention, at least one pharmaceutically acceptable excipient is selected from the group comprising binders, disintegrants, lubricants, glidant or mixtures thereof.

According to an embodiment of the present invention, binder can be used in the formulation. Suitable binders are selected from the group comprising hydroxypropyl cellulose, sodium carboxymethyl cellulose, polyvinylpyrrolidone, sodium carboxymethyl cellulose, polyethylene glycol, starch, pregelatinized starch, sodium alginate, hydroxypropyl methyl cellulose, carboxy methyl cellulose, polymethacrylates, methacrylate polymers, polydextrose, polyethylene oxide or mixtures thereof.

According to an embodiment of the present invention, the binder is hydroxypropyl cellulose.

According to an embodiment of the present invention, the amount of binders is 0.5% to 4.0% by weight in the total composition.

Suitable lubricant is selected from the group comprising talc, sodium stearyl fumarate, magnesium stearate, potassium stearate, stearic acid, sodium chloride, sodium benzoate, sodium acetate, sodium oleate, polyethylene glycols or mixtures thereof.

According to one embodiment of the present invention, the lubricant is magnesium stearate. It helps to provide the desired flowability and compressibility.

According to one embodiment of the present invention, the amount of lubricant is between 0.5% and 3.0% by weight in the total formulation.

According to one embodiment of the present invention, glidant can be used in the formulation. Suitable glidants are selected from the group comprising anhydrous colloidal silicon dioxide, hydrophilic polymers, silicon dioxide or mixtures thereof.

According to one embodiment of the present invention, the glidant is anhydrous colloidal silicon dioxide.

According to one embodiment of the present invention, the amount of glidant is between 0.1% and 1.5% by weight in the total formulation.

Brivaracetam is stable to heat exposure but degrades and picks up water when exposed to both high temperature and humidity however, this problem is minimized by using film coating with appropriate content. Thus, stability is ensured.

According to one embodiment of the present invention, the film coated is based on Polyvinyl Alcohol or Hydroxypropylmethyl cellulose.

According to one embodiment of the present invention, the amount of film coated is between 2.0% and 5.0% by weight in the total formulation. This help to affect positively the distribution time.

In direct compression and dry granulation, the solid pharmaceutical compositions ingredients are not exposed to moisture, solvents and heat. Thus, in this invention, direct compression or dry granulation is used to process moisture, solvent and/or heat sensitive brivaracetam. In addition, these methods provides the desired compressibility and flowability without loss of active substance.

### Example 1: The film coated tablet

| **Ingredients** | **% by weight** | **% by weight** |
|---|---|---|
| Brivaracetam | 8.8 | 17.62 |
| Microcrystalline cellulose | 38.07 | 32.6 |
| Lactose Anhydrous | 42.15 | 38.8 |
| Croscarmellose Sodium | 4.7 | 4.7 |
| Colloidal silicon dioxide | 0.47 | 0.47 |
| Mg Stearate | 1.05 | 1.05 |
| Film-coating | 4.76 | 4.76 |
| **Total** | **100** | **100** |

A process for example 1;
a) Mixing weighed and sieved Brivaracetam, Microcrystalline cellulose, Lactose Anhydrous, croscarmellose sodium, Colloidal silicon dioxide for 15 minutes,
b) Adding weighed and 0.6 mm sieved Magnesium stearate to dry mixture and mixing for 3 minutes,
c) Compressing the mixture into tablets at appropriate specifications,
d) Coating the tablets with film coating.

### Example 2; The film coated tablet

| **Ingredients** | **% by weight** | **% by weight** |
|---|---|---|
| Brivaracetam | 8.94 | 17.89 |
| Microcrystalline cellulose | 39.06 | 33.43 |
| Lactose Monohydrate | 42.7 | 39.4 |
| Sodium Starch Glycolate | 4.85 | 4.83 |
| Mg Stearate | 1.07 | 1.07 |
| Film-coating | 3.38 | 3.38 |
| **Total** | **100** | **100** |

**A process for example 2;**
a) Mixing weighed and sieved Brivaracetam, Lactose monohydrate, Microcrystalline cellulose, Sodium Starch Glycolate for 15 minutes,
b) Adding weighed and 0.6 mm sieved Magnesium stearate to dry mixture and mixing for 3 minutes,
c) Compressing the mixture into tablets at appropriate specifications,
d) Coating the tablets with film coating.

### Example 3; The film coated tablet

| **Ingredients** | **% by weight** | **% by weight** |
|---|---|---|
| Brivaracetam | 8.90 | 17.8 |
| Microcrystalline cellulose | 50.8 | 44.8 |
| HPC | 1.7 | 1.7 |
| HPC-L | 9.85 | 9.8 |
| Starch | 24.3 | 21.45 |
| Mg Stearate | 1.07 | 1.07 |
| Film-coating | 3.38 | 3.38 |
| **Total** | **100** | **100** |

**A process for example 3;**
a) Mixing weighed and sieved Brivaracetam, Microcrystalline cellulose, HPC, HPC-L, Starch for 15 minutes,
b) Adding weighed and 0.6 mm sieved Magnesium stearate to dry mixture and mixing for 3 minutes
c) Compressing the mixture into tablets at appropriate specifications,
d) Coating the tablets with film coating.

### Example 4; The film coated tablet

| **Ingredients** | **% by weight** | **% by weight** |
|---|---|---|
| Brivaracetam | 8.90 | 17.80 |
| Talc | 31.5 | 28.23 |
| Microcrystalline cellulose | 50.1 | 44.72 |
| Croscarmellose Sodium | 4.8 | 4.8 |
| Mg Stearate | 1.07 | 1.07 |
| Film-coating | 3.38 | 3.38 |
| **Total** | **100** | **100** |

**A process for example 4;**
a) Mixing weighed and sieved Brivaracetam, Talc, Microcrystalline cellulose, Croscarmellose Sodium for 15 minutes,
b) Adding weighed and 0.6 mm sieved Magnesium stearate to dry mixture and mixing for 3 minutes,
c) Compressing the mixture into tablets at appropriate specifications,
d) Coating the tablets with film coating.

### Film coating examples

**1)**

| |
|---|
| Polyvinyl Alcohol |
| Titanium dioxide |
| *Talc* |
| Macrogol 3350 |
| Coloring agent |

**2)**

| |
|---|
| Hypromellose |
| Titanium dioxide |
| Macrogol 3350 |
| Coloring agent |

## Claims

1. A film coated tablet comprises brivaracetam and at least one pharmaceutically acceptable excipient wherein microcrystalline cellulose as diluent is present and the amount of disintegrant is 4.0% to 10.0% by weight in the total composition.

2. The film coated tablet according to claim 1, wherein the amount of microcrystalline cellulose is 25.0% to 50.0% by weight in the total composition.

3. The film coated tablet according to claim 1, wherein disintegrants are selected from the group comprising crospovidone, croscarmellose sodium, low-substituted hydroxypropyl cellulose, sodium starch glycolate, carboxymethyl cellulose, sodium carboxymethyl cellulose, calcium carboxymethyl cellulose, sodium carboxymethyl starch, hydroxymethyl starch or mixtures thereof.

4. The film coated tablet according to claim 1, wherein the disintegrant is croscarmellose sodium or low-substituted hydroxypropyl cellulose or sodium starch glycolate or mixtures thereof.

5. The film coated tablet according to claim 1, wherein the film coated tablet comprises one more diluent which are selected from the group comprising lactose anhydrous, lactose, lactose monohydrate, corn starch, starch, mannitol, talc, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

6. The film coated tablet according to claim 5, wherein the diluent is talc, starch, lactose monohydrate or lactose anhydrous or mixtures thereof.

7. The film coated tablet according to claim 5, wherein the amount of diluents is 70.0% to 86.0% by weight in the total composition.

8. The film coated tablet according to claim 1, wherein the amount of brivaracetam is 5.0% to 20.0% by weight in the total composition.

9. The film coated tablet according to claim 1, wherein brivaracetam has a d (0.9) particle size between 100 µm to 300 µm.

10. The film coated tablet according to claim 1, wherein brivaracetam has a d (0.1) particle size between 1 µm to 40 µm or between 4 µm to 34 µm or between 6 µm to 25 µm.

11. The film coated tablet according to claim 1, wherein brivaracetam has a d (0.5) particle size between 8 µm to 85 µm or between 13 µm to 76 µm or between 22 µm to 65 µm or between 27 µm to 58 µm.

12. The film coated tablet according to claim 1, wherein brivaracetam has a d (0.1) particle size between 1 µm to 40 µm and a d (0.5) particle size between 8 µm to 85 µm and a d (0.9) particle size between 100 µm to 300 µm.

13. The film coated tablet according to claim 1, wherein at least one pharmaceutically acceptable excipient is selected from the group comprising binders, disintegrants, lubricants, glidant or mixtures thereof.

14. The film coated tablet according to claim 13, wherein binders are selected from the group comprising hydroxypropyl cellulose, sodium carboxymethyl cellulose, polyvinylpyrrolidone, sodium carboxymethyl cellulose, polyethylene glycol, starch, pregelatinized starch, sodium alginate, hydroxypropyl methyl cellulose, carboxy methyl cellulose, polymethacrylates, methacrylate polymers, polydextrose, polyethylene oxide or mixtures thereof.

15. The film coated tablet according to claim 1, wherein the film coated is based on Polyvinyl Alcohol or Hydroxypropylmethyl cellulose.
